# EUROPÄISCHE PATENTSCHRIFT

(11) **EP 0 721 951 B1**
(45) Veröffentlichungstag und Bekanntmachung des Hinweises auf die Patenterteilung: **02.05.2002**
(21) Anmeldenummer: 95120590.5
(22) Anmeldetag: 27.12.1995
(51) Int. Cl.: C07F 5/00

(54) **Komplexverbindungen der Lanthanoiden mit heterocyclischen Carbenen**
Complexes of lanthanoides with heterocyclic carbenes
Complexes de lanthanoides avec des carbènes hétérocycliques

(30) Priorität: 29.12.1994 DE 4447070
(43) Veröffentlichungstag der Anmeldung: 17.07.1996
(73) Patentinhaber: Celanese Chemicals Europe GmbH, 60439 Frankfurt am Main (DE)
(72) Erfinder: Herrmann, Wolfgang A., Prof. Dr., D-85354 Freising (DE)

(56) Entgegenhaltungen:
- ANGEWANDTE CHEMIE, INTERNATIONAL EDITION ENGLISH, Bd. 33, Nr. 17, 1994, Seiten 1733-1734, XP002001031 SCHUMANN, H. ET AL.: "ORGANOLANTHANOID-CARBENE ADDUCTS"
- JOURNAL OF THE AMERICAN CHEMICAL SOCIETY, Bd. 116, 1994, Seiten 7927-7928, XP002001032 ARDUENGO, A.J. III. ET AL.: "CARBENE-LANTHANIDE COMPLEXES"
- CHEMISCHE BERICHTE, Bd. 127, 1994, Seiten 2369-2372, XP002001033 SCHUMANN, H. ET AL.: "CARBEN-ADDUKTE DES ZWEIWERTIGEN SAMARIUMS UND YTTERBIUMS"

## Beschreibung

Die Erfindung betrifft Komplexverbindungen der Elemente mit den Ordnungszahlen 57 bis 71, ausgenommen Promethium, mit heterocyclischen Carbenen als Komplexliganden. Die Gruppe der Seltenen Erden umfaßt die 14 Elemente Cer, Praseodym, Neodym, Promethium, Samarium, Europium, Gadolinium, Terbium, Dysprosium, Holmium, Erbium, Thulium, Ytterbium und Lutetium ("Seltenerdmetalle"). Aufgrund großer chemischer Ähnlichkeiten faßt man sie mit den Metallen der dritten Nebengruppe (Gruppe 3 nach der IUPAC-Empfehlung 1985), nämlich Scandium, Yttrium und Lanthan, unter dem Namen Lanthanoidelemente zusammen.

In ihren chemischen Verbindungen sind die 17 Elemente dieser Gruppe vorwiegend dreiwertig, jedoch bilden einzelne Vertreter der Lanthanoiden Verbindungen, in denen sie vierwertig (z.B. Cer, Terbium) und zweiwertig (z.B. Europium, Ytterbium) sind. Die chemischen Verbindungen der Lanthanoide sind durch vorwiegend ionische Bindungsverhältnisse und eine ausgesprochene Neigung zur Bindung an Sauerstoff geprägt. Die Anwendungen der Lanthanoid-Verbindungen sind sehr vielfältig und leiten sich in erster Näherung von der Lewis-Acidität der Metall-Ionen, insbesondere in den höheren Oxidationsstufen, sowie von den elektrochemischen Eigenschaften als Reduktions- und Oxidationsmittel ab. So ist vierwertiges Cer ein kräftiges Einelektronen-Oxidationsmittel. Zweiwertiges Samarium wird in der organischen Synthese als selektiv wirkendes Kupplungsreagenz verwendet. Viele Lanthanoide eignen sich zur Dotierung von Glühlampenmaterialien, weil sie die Elektronen-Austrittsarbeit aus Metall-Legierungen stark erniedrigen.

Nahezu alle Lanthanoid-Ionen sind zur Solvatation befähigt, d.h. zur Anlagerung von Lewis-basischen Liganden (z.B. Ether, Amine). Diese Basenanlagerungen führen häufig zu verbesserter Löslichkeit der Lanthanoid-Verbindungen in organischen Lösemitteln. Gleichzeitig wird die hydrolytische Stabilität erhöht, die bei einfachen Lanthanoid-Verbindungen wie den Halogeniden gering ist. Überdies trifft auch eine oft vorteilhafte Erniedrigung der Lewis-Acidität der betreffenden Metall-Ionen ein. Berücksichtigt man, daß zahlreiche Lanthanoid-Verbindungen als Katalysatoren bei Reaktionen organischer Verbindungen wirksam sind, dann ist die gezielte Steuerung der Metall-Reaktivität der Lanthanoide besonders wünschenswert. Bedeutung haben in diesem Zusammenhang ausreichend nucleophile Liganden, die stabil, d.h. ohne Ausbildung der üblichen Dissoziationgleichgewichte, an die Lanthanoid-Ionen gebunden sind. Gleichzeitig sollen sie sich konstitutionell so vielfältig variieren lassen, daß sie gezielt als stereoelektronische Steuerliganden für die Reaktivität der Lanthanoide eingesetzt werden können. Solche Steuerliganden müssen oxidationsbeständig sein und sollen mit anderen an die Lanthanoid-Ionen gebundenen Gruppen nicht reagieren. Außerdem ist es erforderlich, daß diese Liganden die Löslichkeit der Lanthanoid-Verbindungen, an welche sie gebunden sind, in organischen Lösemitteln erhöhen. Schließlich dürfen sie keine über die Komplexbildung hinausgehenden Veränderungen durch das Lanthanoid-Ion erfahren.

Da die meisten Lanthanoid-Ionen "harte Kationen" im Sinne des Pearson-Konzepts (vgl. z.B. Römpp, Chemie-Lexion 1987, Bd. 5, Seite 3651 ff) sind, bevorzugen diese zwei-, dreiund vierwertigen Metall-Ionen entsprechend "harte Basen" als Liganden in der Chemie der Lanthanoide. Den elektronisch equivalenten Thioethern und Phosphinen kommt dagegen nur untergeordnete Bedeutung zu, weil sie als "weiche Lewis-Basen" deutlich schwächer an die Seltenerdmetall-Ionen koordinativ gebunden sind. Außerdem unterliegen sie relativ leicht Oxidationsprozessen, die zur Bildung von Sulfonen, Schwefeldioxiden bzw. Phosphinoxiden führen.

Es bestand daher die Aufgabe Komplexverbindungen der Lanthanoide bereitzustellen, die koordinativ fest gebundene Liganden enthalten und beständig gegen Oxidationsvorgänge sind.

Diese Aufgabe wird gelöst durch Komplexverbindungen der allgemeinen Formel

[LₐLn_{b}X_{c}]ⁿ(A)ₙ (I)

in der Ln für Ionen in den Oxidationsstufen 2, 3 oder 4 der Lanthanoide (Elemente mit den Ordnungszahlen 57 bis 71 im Periodensystem der Elemente) ausgenommen Europium und Promethium als Zentralatom steht, X an das Zentralatom gebundene ein- oder mehrzähnige geladene oder ungeladene Liganden bedeutet und L ebenfalls an das Zentralatom gebundene Monocarbene der allgemeinen Formeln 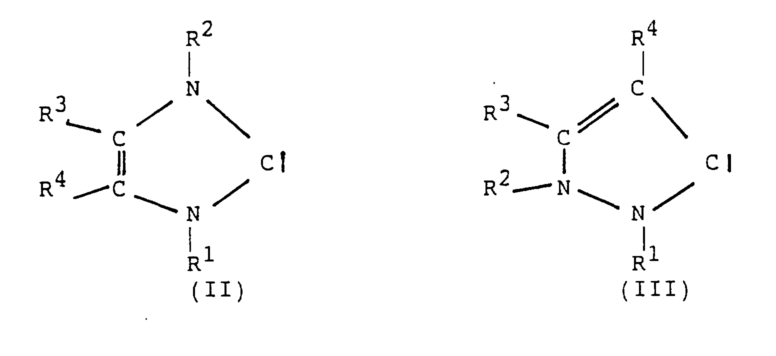 oder Dicarbene der allgemeinen Formeln 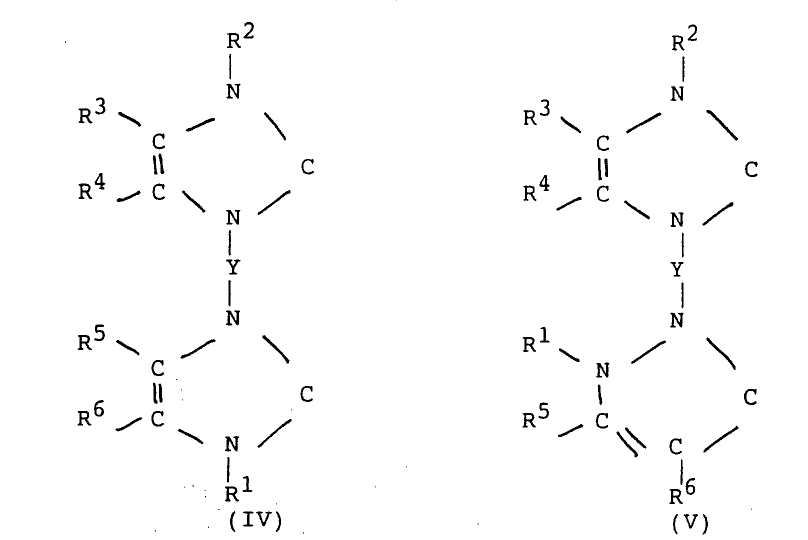 sind, wobei R¹, R², R³, R⁴, R⁵ und R⁶ gleiche oder verschiedene geradkettige oder verzweigte, gegebenenfalls sulfonierte Alkylreste mit 1 bis 7 Kohlenstoffatomen, gegebenenfalls sulfonierte aliphatische mono- oder polycyclische Reste mit 5 bis 18 Kohlenstoffatomen, gegebenenfalls sulfonierte Alkenylreste mit 2 bis 5 Kohlenstoffatomen, gegebenenfalls sulfonierte Arylreste mit 6 bis 14 Kohlenstoffatomen oder gegebenenfalls sulfonierte Arylalkylreste mit 7 bis 19 Kohlenstoffatomen bedeuten, R³, R⁴, R⁵ und R⁶ auch für Wasserstoff stehen können, R³ und R⁴ gemeinsam sowie R⁵ und R⁶ gemeinsam jeweils auch gleiche oder verschiedene anellierte und gegebenenfalls sulfonierte Reste mit 3 bis 7 Kohlenstoffatome bedeuten können, Y ein gesättigter oder ungesättigter, geradkettiger oder verzweigter Alkylidenrest mit 1 bis 4 Kohlenstoffatomen oder ein Dialkylsilylen- oder ein Tetraalkyldisilylenrest ist, A für ein einfach geladenes Anion oder das chemische Äquivalent eines mehrfach geladenen Anions steht, b eine ganze Zahl von 1 bis 4 darstellt, a einer ganzen Zahl von 1 bis 4 · b und c null oder einer ganzen Zahl von 1 bis 4 · b entsprechen und n null oder eine ganze Zahl von 1 bis 3 · b ist, mit der Maßgabe, daß X nicht für den Cyclopentadienyl- oder für den substituierten Cyclopentadienylrest, für den Acetylacetonat- oder für den substituierten Acetylacetonatrest steht.

In der Literatur finden sich bisher nur zwei Hinweise auf Komplexverbindungen der Lanthanoide mit Liganden, die sich von heterocyclischen Carbenen ableiten. Die Carbene sind hierbei an die Molekülfragmente Bis (η⁵-pentamethyl-cyclopentadienyl)ytterbium bzw. -samarium gebunden. Diese Komplexverbindungen enthalten in Form der Pentadienylreste stark elektronenliefernde π-Liganden, die mit den herkömmlichen Metallhalogeniden, -alkoxiden und -amiden chemisch und strukturell nicht vergleichbar sind (Arduengo et al., J.Am.Chem.Soc. 1994, 116, 7927 ff; Schumann et al., Angew. Chem. 1994, 106, 1829 ff).

Im Hinblick auf diesen Stand der Technik ist es überraschend, daß sich von Stickstoff-Heterocyclen abgeleitete Carbene als stabil gebundene Liganden an zwei-, drei- und vierwertige Lanthanoid-Ionen koordinieren lassen, ohne daß zusätzliche andere organische Liganden wie Cyclopentadienyl oder Pentamethylcyclopentadienyl anwesend sind.

Die neuen Verbindungen sind in organischen Lösungsmitteln, aber auch in Wasser löslich, insbesondere wenn sie durch Sulfonatreste substituierte aliphatische oder aromatische Reste enthalten. Sie sind thermisch sehr beständig und zeichnen sich durch hohe Oxidationsstabilität aus.

Die neuen Verbindungen leiten sich von den Elementen Scandium, Yttrium, Lanthan, Cer, Praseodym, Neodym, Samarium, Gadolinium, Terbium, Dysprosium, Holmium, Erbium, Thulium, Ytterbium und Lutetium in den Oxidationsstufen zwei, drei und vier ab. Promethium als radioaktives Element wird im Rahmen der vorliegenden Erfindung aus der Gruppe der Lanthanoiden ausgenommen, ebenso Europium als den Erdalkalimetallen Strontium und Barium sehr ähnliches Element.

Ein- oder mehrzähnige Liganden, die neben den Carbenen in den Komplexverbindungen enthalten sein können und in der allgemeinen Formel (I) durch X wiedergegeben werden, sind Wasserstoff oder das Wasserstoff-Ion, Halogene oder Halogenide, Pseudohalogenide, Carboxylat-Ionen, Sulfonat-Ionen, Amidreste, Alkoholatreste, Kohlenmonoxid, Stickstoffmonoxid, Nitrile, Isonitrile, Mono- oder Diolefine, Alkine und π-Aromatenreste. Sind mehrere dieser Liganden im Komplexmolekül enthalten, dann können sie gleich oder verschieden sein.

In den vom Imidazol und vom Pyrazol oder deren Derivaten abgeleiteten Mono- bzw. Dicarbenen entsprechend den Formeln (II), (III), (IV) und (V) stehen R¹ bis R⁶ insbesondere für die Reste Methyl, Isopropyl, tert.-Butyl, Benzyl, Triphenylmethyl, Phenyl, Tolyl, Mesityl und Adamantyl, R¹ und R² sind bevorzugt der Methyl-, der tert.-Butyl-, der Phenyl-, der Benzylrest und der o-Tolylrest. R³ und R⁴ stehen vorzugsweise für Wasserstoff und die Methylgruppe.

Die Reste R³ und R⁴ und die Reste R⁵ und R⁶ können zusammen mit zwei benachbarten Kohlenstoffatomen des Imidazolringes oder des Pyrazolrings ein Ringsystem bilden. R³ und R⁴ bzw. R⁵ und R⁶ stehen dann bevorzugt für die Gruppierungen (CH)₄, sie führt zur Ausbildung eines anellierten aroamtischen Sechsrings, (CH₂)₄ und (CH₂)₅.

Die durch Y bezeichneten Brückenglieder der Dicarbene gemäß den Formeln (IV) und (V) sind vorzugsweise die Methylen-, Dimethylmethylen-, Diphenylmethylen-, 1,3-Phenylen- und die Ethylidengruppe. Unter den Silizium enthaltenden Brückengliedern werden die Dimethylsilylen- und die Tetramethyldisilylengruppe bevorzugt.

In der allgemeinen Formel (I) ist b vorzugsweise 1; A vertritt bevorzugt Halogenid-, Pseudohalogenid-, Tetraphenylborat-, Tetrafluoroborat-, Hexafluorophosphat- und Carboxylat-Ionen, unter den zuletzt genannten insbesondere das Acetat-Ion, ferner Metallkomplex-Anionen wie z.B. Tetracarbonylcobaltat, Hexafluoroferrat(III), Tetrachloroferrat, Tetrachloroaluminat oder Tetrachloropalladat (II).

Die beanspruchten Verbindungen sind auf verschiedenen Wegen zugänglich. Nach einer Herstellungsvariante setzt man freies Carben, das aus dem zugehörigen Azolium-Salz durch Deprotonierung erhalten wurde, mit einer Lanthanoid-Verbindung um. Das Carben kann zuvor in Substanz isoliert worden sein oder es wird in situ hergestellt. Geeignete Lanthanoid-Verbindungen sind die Halogenide, Carboxylate, Acetylacetonate, Trifluormethansulfonate, ferner die Alkoxide, Phenolate, die Mono- und Dialkylamide und die Mono- und Diphenylamide. Alkoxide und Alkylamide enthalten Alkylreste mit 1 bis 5 Kohlenstoffatomen, die im Falle der Dialkylamide gleich oder verschieden sein können.

Ein weiterer Weg die neuen Komplexverbindungen herzustellen, ist die unmittelbare Umsetzung einer Lanthanoid-Verbindung mit einem Azoliumsalz, d.h. ohne vorherige Bildung eines Carbens aus dem Azolium-Salz. Die Lanthanoid-Verbindungen lassen sich durch die allgemeine Formel LnZₘ (m= 2, 3 oder 4) beschreiben, in der Z für Halogenid, Pseudohalogenid, Carboxylat, Acetylacetonat, Alkoxid (mit C₁ bis C₅-Alkylresten), Phenolat, Mono- und Dialkylamid (mit C₁ bis C₅-Alkylresten, die im Falle des Diamids gleich oder verschieden sein können), Disilylamid steht. Die Azolium-Salze entsprechen der allgemeinen Formen [L-H]Z oder [L-H]A wobei L, Z und A die bereits beschriebenen Bedeutungen haben.

Die Umsetzung der Lanthanoid-Verbindungen mit den Carbenen und gegebenenfalls weiteren Liganden erfolgt durch Mischen der Reaktanten in einem Lösungsmittel bei niedrigen Temperaturen (z.B. -78 °C), bei Raumtemperatur oder bei erhöhter Temperatur. Die Reaktion verläuft mit hoher Geschwindigkeit und ist im wesentlichen oft nach wenigen Minuten beendet. Es empfiehlt sich jedoch zur Vervollständigung der Reaktion Umsetzungszeiten von bis zu mehreren Stunden einzuhalten, insbesondere wenn die Einsatzstoffe im verwendeten Medium nur teilweise gelöst sind, d.h. aus Suspension reagieren.

Zur Herstellung sulfonierter Liganden enthaltender Komplexverbindungen, die wasserlöslich sind, setzt man als Ausgangsstoffe zumindest einen Reaktionspartner ein, dessen Molekül oder Molekülfragment sulfoniert ist.

Zur Isolierung der neuen Komplexverbindungen aus dem Reaktionsmedium hat es sich bewährt, das Lösungsmittel zweckmäßig im Hochvakuum zu entfernen. Das Rohprodukt wird zur Reinigung gewaschen und aus einem geeigneten Lösungsmittel oder Lösungsmittelgemisch, das im Einzelfall durch Vorversuche zu ermitteln ist, umkristallisiert.

Die Lanthanoid-Carben-Komplexe I sind Katalysatoren für Reaktionen, die durch Lewissäuren katalysiert werden, z.B. die Herstellung von Polylactiden (das sind bioabbaubare Polymere), sowie für CH-,CC-,CSi- und NC-Verknüpfungsreaktionen. Hierzu gehören die Hydroaminierung, Hydrierung, Oligomerisierung, Polymerisation von Olefinen, Isomerisierung und Hydrosilylierung von Olefinen, Hydroborierung von Olefinen und Alkinen, die Michael-Reatkion, die Diels-Alder-Reaktion, die Friedel-Crafts-Acylierung von Aromaten und die Addition von Grignard-Reagenzien an 1,3-Diketone.

Nachstehend werden Herstellung und Eigenschaften der neuen Verbindungen beschrieben, die Erfindung ist jedoch nicht auf die angeführten Beispiele beschränkt.

### Beispiel 1: Tris(1,3-dimethylimidazolin-2-yliden)erbiumtrichlorid

(a) Zu 0.508 g (1.0 mmol) ErCl₃(thf)_{3,25}, suspendiert in 50 ml Tetrahydrofuran (THF; thf) werden 22.6 ml einer 0,14 molaren Lösung von 1,3-Dimethylimidazol-2-yliden in THF (entsprechend 3,25 mmol Carben) getropft. Man läßt 3 d bei Raumtemperatur rühren, anschließend filtriert man das Lösungsmittel ab. Nach 5 h Trocknen im Hochvakuum bei 0,1 Pa erhält man 0.392 g (70 %) des analysenreinen, gelben Produktes.
(b) 1 Mol-Äquivalent Er[N(SiMe₃)]₃ (Me: -CH₃) und 3 Mol-Äquivalente 1,3-Dimethylimidazoliumchlorid werden in 50 ml THF suspendiert. Man erhitzt die Suspension 5 d unter Rückfluß. Nach Abdestillieren des Lösungsmittels und 6 h Trocknen im Hochvakuum bei 0,1 Pa erhält man das analysenreine, gelbe Produkt.

### Charakterisierung

C₁₅H₂₄Cl₃ErN₆ (562,02)

| | | | | |
|---|---|---|---|---|
| ber. | C 32,6 | H 4,3 | N 14,94 | Cl 18,92 |
| gef. | C 31,92 | H 4,15 | N 13,98 | Cl 18,98 |

- IR:: ν = 3153 cm⁻¹ s, 3101 s, 1573 s, 1397 vs, 1313 s, 1221 vs, 1174 s, 1113 s, 1076 m, 1018 m, 1003 m 972 m, 938 m, 917 m, 894 w, 740 vs, 648 w, 622 m, 609 w, 450 m.
- MS(EI):: m/2z= 96 a.m.u. (100 %) [Carben], 81 (4) [Carben - Me].

### Beispiel 2: (1,3-Dimethylimidazolin-2-yliden)(tetrahydrofuran)tris[bis(dimethylsilyl) amido]yttrium

Zu 0.756 g (1.2 mmol) Y(bdsa)₃(thf)₂, amid (bdsa:
Bis(dimethylsilyl)amid) in 35 ml THF werden langsam 4.2 ml einer 0,3 molaren Lösung von 1,3-Dimethyl-imidazol-2-yliden in THF (entsprechend 1,2 mmol Carben) getropft. Man rührt 24 h bei Raumtemperatur, destilliert das Lösungsmittel ab und extrahiert das Produkt mit 20 ml n-Hexan. Nach dem Trocknen erhält man 0.776 g (99 %) des grünlichen Produkts in analysenreiner Qualität.

### Charakterisierung

C₂₁H₅₈N₅OSi₆Y (654,15)

| | | | |
|---|---|---|---|
| ber. | C 38,56 | H 8,94 | N 10,71 |
| gef. | C 38,37 | H 8,61 | N 10,56 |

- IR:: ν= 3169 cm⁻¹ w, 3135 w, 2070 vs, 1992 (sh)m, 1927 (sh)m, 1773 w, 1540 w, 1401 m, 1316 m, 1243 vs, 1220 m, 1169 w, 1154 w, 1112 m, 1048 (br)vs, 970 vs 941 vs, 898 (br)vs, 836 vs, 788 vs, 763 vs, 724 s, 682 m, 624 w, 609 w, 446 w, 409 w.
- ¹H-NMR (C₆D₆):: δ= 0.35 ppm (36H, s, SiCH₃), 1.41 (4H, d, THF), 3.38 (6H, s, NCH₃), 3.56 (4H, d, THF), 5.04 (6H, SiH) 5.79 (2H, s, CH).
- ⁻¹³C{¹H}-NMR (C₆D₆):: δ= 3.2 ppm (q, SiCH₃), 25.7 (t, THF), 37.5 (q, NCH₃), 67.9 (t, THF), 121.1 (d, CH), 168.2 (s, NCN).

### Beispiel 3: trans-Bis(1,3-dimethylimidazolin-2-yliden) tris[bis(dimethylsilyl)amido]yttrium

Zu 0,756 g (1.2 mmol) Y(bdsa)₃(thf)₂ (bdsa: siehe Beispiel 2), gelöst in 35 ml THF, werden langsam 8.7 ml einer 0,3 molaren Lösung von 1,3-Dimethylimidazol-2-yliden in THF (entsprechend 2,4 mmol Carben) getropft. Man rührt 24 h bei Raumtemperatur, destilliert das Lösungsmittel ab und extrahiert das Produkt mit 20 ml n-Hexan. Nach dem Trocknen erhält man 0.805 g (99 %) hellbraunes Produkt in analysenreiner Qualität.

### Charakterisierung

C₂₂H₅₈N₇Si₆Y (678,18)

| | | | |
|---|---|---|---|
| ber. | C 38,96 | H 8,62 | N 14,46 |
| gef. | C 38,78 | H 8,70 | N 14,50 |

- IR:: ν= 3163 cm ¹ w, 3130 w, 2088 m, 2040 (sh)m, 1532 w, 1400 m, 1312 w, 0,26 ppm (1247 s, 1240 s, 1216 m, 1169 w, 1155 w, 1104 w, 1032 s, 969 (sh)m, 936 s, 893 vs, 834 s, 781 s, 760 s, 728 s, 720 s, 693 w, 675 w, 615 w, 602 w, 445 w, 433 w.
- ¹H-NMR (C₆D₆):: δ= 0.26 ppm (36H, s, SiCH₃), 3.70 (12H, s, NCH₃), 5.10 (6H, sep., SiH), 6.03 (4H, s, CH).
- ¹³C-NMR(C₆D₆):: δ= 3.5 ppm (SiCH₃), 38.6 (NCH₃), 120.7 (CH), (NCN) nicht beobachtet.

## Patentansprüche

1. Komplexverbindungen der allgemeinen Formel
[LₐLn_{b}X_{c}]ⁿ(A)ₙ (I)
in der Ln für Ionen in den Oxidationsstufen 2, 3 oder 4 der Lanthanoide (Elemente mit den Ordnungszahlen 57 bis 71 im Periodensystem der Elemente) ausgenommen Europium und Promethium als Zentralatom steht, X an das Zentralatom gebundene ein- oder mehrzähnige geladene oder ungeladene Liganden bedeutet und L ebenfalls an das Zentralatom gebundene Monocarbene der allgemeinen Formeln 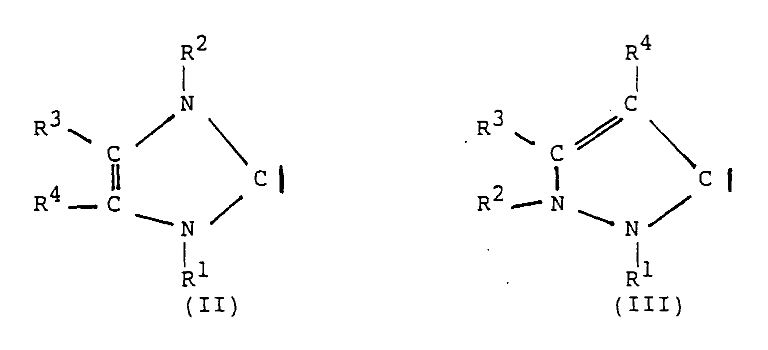 oder Dicarbene der allgemeinen Formeln 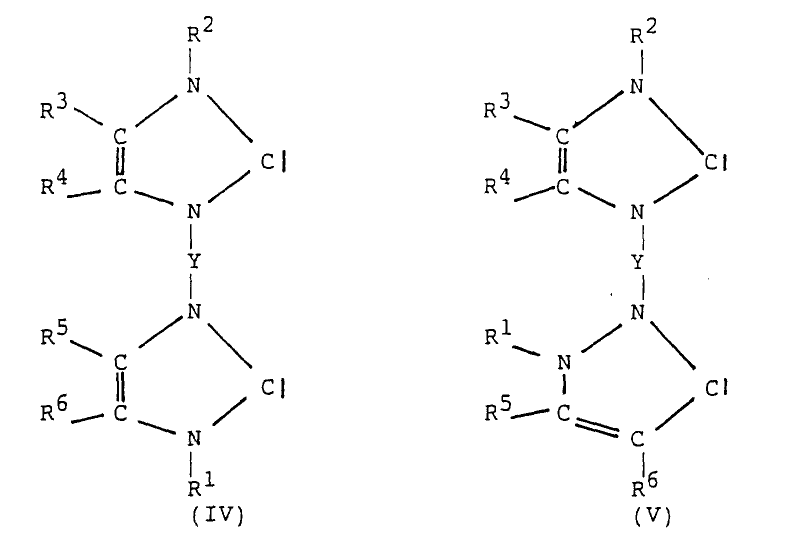 sind, wobei R¹, R², R³, R⁴, R⁵ und R⁶ gleiche oder verschiedene geradkettige oder verzweigte, gegebenenfalls sulfonierte Alkylreste mit 1 bis 7 Kohlenstoffatomen, gegebenenfalls sulfonierte aliphatische mono- oder polycyclische Reste mit 5 bis 18 Kohlenstoffatomen, gegebenenfalls sulfonierte Alkenylreste mit 2 bis 5 Kohlenstoffatomen, gegebenenfalls sulfonierte Arylreste mit 6 bis 14 Kohlenstoffatomen oder gegebenenfalls sulfonierte Arylalkylreste mit 7 bis 19 Kohlenstoffatomen bedeuten, R³, R⁴, R⁵ und R⁶ auch für Wasserstoff stehen, R³ und R⁴ gemeinsam sowie R⁵ und R⁶ gemeinsam jeweils auch gleiche oder verschiedene anellierte und gegebenenfalls sulfonierte Reste mit 3 bis 7 Kohlenstoffatome bedeuten, Y ein gesättigter oder ungesättigter, geradkettiger oder verzweigter Alkylidenrest mit 1 bis 4 Kohlenstoffatomen oder ein Dialkylsilylen- oder ein Tetraalkyldisilylenrest ist, A für ein einfach geladenes Anion oder das chemische Äquivalent eines mehrfach geladenen Anions steht, b eine ganze Zahl von 1 bis 4 dar stellt, a einer ganzen Zahl von 1 bis 4 · b und c null oder einer ganzen Zahl von 1 bis 4 · b entsprechen und n null oder eine ganze Zahl von 1 bis 3 b ist, mit der Maßgabe, daß X nicht für den Cyclopentadienyl- oder für den substituierten Cyclopentadienylrest, für den Acetylacetonat- oder für den substituierten Acetylacetonatrest steht.

2. Komplexverbindungen nach Anspruch 1 **dadurch gekennzeichnet, daß** X in der allgemeinen Formel (I) für Wasserstoff, das Wasserstoff-Ion, Halogene, Halogen-Ionen, Pseudohalogenide, Carboxylat-Ionen, Sulfonat-Ionen, Amidreste, Alkoholatreste, Kohlenmonoxid, Stickstoffmonoxid, Nitrile, Isonitrile, Monooder Diolefine, Alkine und π-Aromatenrest steht.

3. Komplexverbindungen nach Anspruch 1 oder 2 **dadurch gekennzeichnet, daß** in den allgemeinen Formeln (II), (III), (IV) und (V) R¹, R², R³, R⁴, R⁵, R⁶ für die Reste Methyl, Isopropyl, tert.-Butyl, Benzyl, Triphenylmethyl, Phenyl, Tolyl, Xylyl, Mesityl und Adamantyl stehen.

4. Komplexverbindungen nach einem oder mehreren der Ansprüche 1 bis 3 **dadurch gekennzeichnet, daß** in den allgemeinen Formeln (II), (III), (IV) und (V) R¹ und R² für den Methyl-, den tert.-Butyl-, den Phenyl, den Benzyl- und o-Tolylrest stehen.

5. Komplexverbindungen nach einem oder mehreren der Ansprüche 1 bis 4 **dadurch gekennzeichnet, daß** in den allgemeinen Formeln (II), (III), (IV) und (V) R³ und R⁴ für Wasserstoff und den Methylrest stehen.

6. Komplexverbindungen nach einem oder mehreren der Ansprüche 1 bis 5 **dadurch gekennzeichnet, daß** in den allgemeinen Formeln (II), (III), (IV) und (V) R³ und R⁴ gemeinsam und R⁵ und R⁶ gemeinsam für die Gruppierungen (CH)₄, (CH₂)₄, (CH₂)₅ stehen.

7. Komplexverbindungen nach einem oder mehreren der Ansprüche 1 bis 6 **dadurch gekennzeichnet, daß** in den allgemeinen Formeln (IV) und (V) Y für die Methylen-, die Dimethylmethylen-, die Diphenylmethylen- und die Ethylidengruppe steht.

8. Komplexverbindungen nach einem oder mehreren der Ansprüche 1 bis 6 **dadurch gekennzeichnet, daß** in den allgemeinen Formeln (IV) und (V) Y für die Dimethylsilylen- und die Tetramethyldisilylengruppe steht.

9. Komplexverbindungen nach einem oder mehreren der Ansprüche 1 bis 8 **dadurch gekennzeichnet, daß** in der allgemeinen Formel (I) b 1 ist.

10. Komplexverbindungen nach einem oder mehreren der Ansprüche 1 bis 9 **dadurch gekennzeichnet, daß** in der allgemeinen Formel (I) A für Halogenid- und Pseudohalogenid-Ionen, das Tetraphenylborat-, das Tetrafluoroborat-, das Hexafluorophosphat-, das Acetat-, das Tetracarbonyl-cobaltat-, das Hexafluoroferrat-, das Tetrachlorferrat-, das Tetrachloroaluminat- und das Tetrachloropalladat-Ion steht.

## Claims

1. A complex of the formula
[LₐLn_{b}X_{c}]ⁿ(A)ₙ (I)
where Ln is an ion in the oxidation state 2, 3 or 4 of the lanthanides (elements having the atomic numbers from 57 to 71 in the Periodic Table of the Elements), with the exception of europium and promethium, as central atom, X are monodentate or multidentate, charged or uncharged ligands bound to the central atom and L are monocarbenes of the formulae 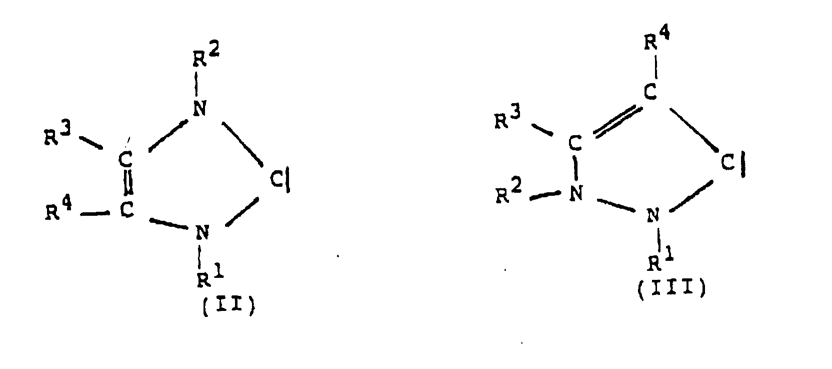 or dicarbenes of the formulae 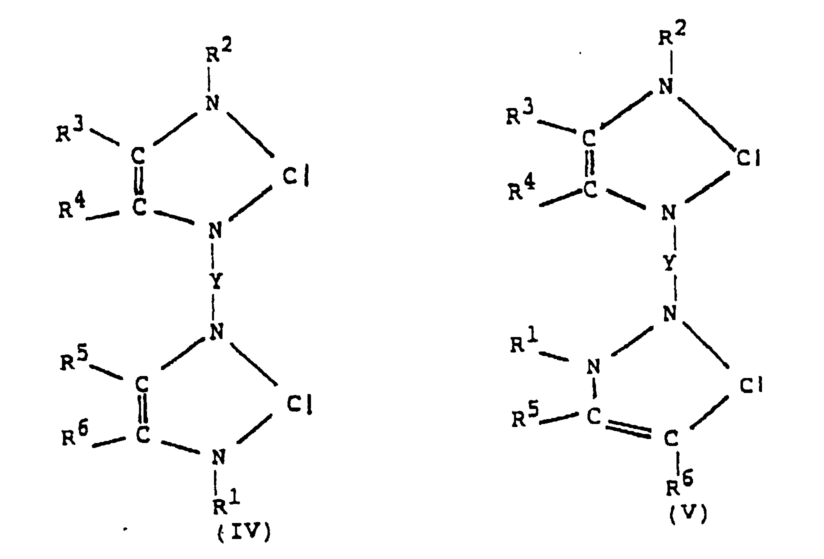 likewise bound to the central atom, where R¹, R², R³, R⁴, R⁵ and R⁶ are identical or different straight-chain or branched, sulfonated or unsulfonated alkyl radicals having from 1 to 7 carbon atoms, sulfonated or unsulfonated aliphatic monocyclic or polycyclic radicals having from 5 to 18 carbon atoms, sulfonated or unsulfonated alkenyl radicals having from 2 to 5 carbon atoms, sulfonated or unsulfonated aryl radicals having from 6 to 14 carbon atoms, or sulfonated or unsulfonated arylalkyl radicals having from 7 to 19 carbon atoms, R³, R⁴, R⁵ and R⁶ can also be hydrogen, R³ together with R⁴ and R⁵ together with R⁶ can in each case also be identical or different fused and sulfonated or unsulfonated radicals having from 3 to 7 carbon atoms, Y is a saturated or unsaturated, straight-chain or branched alkylidene radical having from 1 to 4 carbon atoms or a dialkylsilylene or a tetraalkyldisilylene radical, A is a singly charged anion or the chemical equivalent of a multiply charged anion, b is an integer from 1 to 4, a is an integer from 1 to 4 · b and c is zero or an integer from 1 to 4 · b and n is zero or an integer from 1 to 3 · b, with the proviso, that X does not mean the cyclopentadienyl radical or a substituted cyclopentadienyl radical, the acetylacetonate radical or a substituted acetylacetonate radical.

2. A complex as claimed in claim 1, wherein X in the formula (I) are hydrogen, the hydrogen ion, halogens, halogen ions, pseudohalides, carboxylate ions, sulfonate ions, amide radicals, alkoxide radicals, carbon monoxide, nitrogen monoxide, nitrites, isonitriles, monoolefins or diolefins, alkynes and π-aromatic radicals.

3. A complex as claimed in claim 1 or 2, wherein, in the formulae (II), (III), (IV) and (V), R¹, R², R³, R⁴, R⁵, R⁶ are the radicals methyl, isopropyl, tert-butyl, benzyl, triphenylmethyl, phenyl, tolyl, xylyl, mesityl and adamantyl.

4. A complex as claimed in one or more of claims 1 to 3, wherein, in the formulae (II), (III), (IV) and (V), R¹ and R² are the methyl, the tert-butyl, the phenyl, the benzyl and o-tolyl radicals.

5. A complex as claimed in one or more of claims 1 to 4, wherein, in the formulae (II), (III), (IV) and (V), R³ and R⁴ are hydrogen and the methyl radical.

6. A complex as claimed in one or more of claims 1 to 5, wherein, in the formulae (II), (III), (IV) and (V), R³ together with R⁴ and R⁵ togehter with R⁶ are the moieties (CH)₄, (CH₂)₄, (CH₂)₅.

7. A complex as claimed in one or more of claims 1 to 6, wherein, in the formulae (IV) and (V), Y is the methylene, the dimethylmethylene, the diphenylmethylene and the ethylidene group.

8. A complex as claimed in one or more of claims 1 to 6, wherein, in the formulae (IV) and (V), Y is the dimethylsilylene and the tetramethyldisilylene group.

9. A complex as claimed in one or more of claims 1 to 8, wherein, in the formula (I), b is 1.

10. A complex as claimed in one or more of claims 1 to 9, wherein, in the formula (I), A is a halide or pseudohalide ion, the tetraphenylborate, the tetrafluoroborate, the hexafluorophosphate, the acetate, the tetracarbonylcobaltate, the hexafluoroferrate, the tetrachloroferrate, the tetrachloroaluminate or the tetrachloropalladate ion.

## Revendications

1. Complexes de formule générale
[LₐLn_{b}X_{c}]ⁿ(A)ₙ (I)
dans laquelle Ln représente des ions aux stades d'oxydation 2, 3 ou 4 des lanthanoïdes (éléments de numéros atomiques 57 à 71 de la Classification Périodique) à l'exception de l'europium et du prométhéum pour l'atome central, X représente des ligands chargés ou non, mono- ou polydentiques, fixés sur l'atome central et L représente des monocarbènes, également fixés sur l'atome central, de formules générales 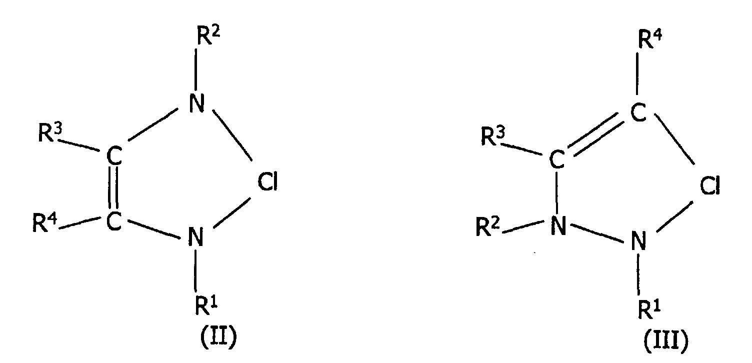 ou des dicarbènes de formules générales 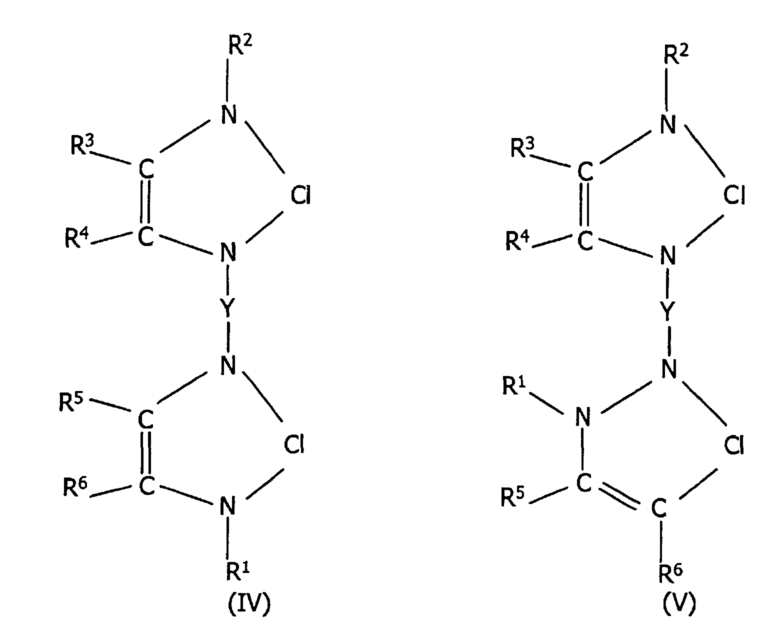 dans lesquelles R¹, R², R³, R⁴, R⁵ et R⁶, ayant des significations identiques ou différentes, représentent chacun un groupe alkyle à chaîne droite ou ramifiée en C₁-C₇ éventuellement sulfoné, un groupe aliphatique mono- ou poly-cyclique en C₅-C₁₈ éventuellement sulfoné, un groupe alcényle en C₂-C₅ éventuellement sulfoné, un groupe aryle en C₆-C₁₄ éventuellement sulfoné ou un groupe arylalkyle en C₇-C₁₉ éventuellement sulfoné, R³, R⁴, R⁵ et R⁶ pouvant également représenter l'hydrogène, R³ et R⁴ d'une part, R⁵ et R⁶ d'autre part, former ensemble des groupes identiques ou différents, condensés et éventuellement sulfonés, en C₃-C₇, Y un groupe alkylidène saturé ou insaturé, à chaîne droite ou ramifiée en C₁-C₄, ou un groupe dialkylsilylène ou tétraalkyldisilylène, A représente un anion à une charge ou l'équivalent chimique d'un anion à plusieurs charges, b est un nombre entier allant de 1 à 4, a est un nombre entier allant de 1 à 4 b, c est égal à zéro ou à un nombre entier allant de 1 à 4 · b et n est égal à zéro ou à un nombre entier allant de 1 à 3 · b, sous réserve que X ne peut représenter un groupe cyclopentadiényle ou cyclopentadiényle substitué, un groupe acétylacétonate ou un groupe acétylacétonate substitué.

2. Complexes selon la revendication 1, **caractérisés en ce que**, dans la formule (I), X représente l'hydrogène, l'ion hydrogène, des halogènes, des ions halogène, des pseudo-halogénures, des ions carboxylate, des ions sulfonate, des radicaux d'amidures, des radicaux d'alcoolates, le monoxyde de carbone, le monoxyde d'azote, des nitriles, des isonitriles, des mono- ou di-oléfines, des alcynes ou un radical de dérivé π-aromatique.

3. Complexes selon la revendication 1 ou 2, **caractérisés en ce que**, dans les formules générales (II), (III), (IV) et (V), R¹, R², R³, R⁴, R⁵, R⁶ représentent des groupes méthyle, isopropyle, tert-butyle, benzyle, triphénylméthyle, phényle, tolyle, xylyle, mésityle et adamantyle.

4. Complexes selon une ou plusieurs des revendications 1 à 3, **caractérisés en ce que**, dans les formules générales (II), (III), (IV) et (V), R¹ et R² représentent des groupes méthyle, tert-butyle, phényle, benzyle ou o-tolyle.

5. Complexes selon une ou plusieurs des revendications 1 à 4, **caractérisés en ce que**, dans les formules générales (II), (III), (IV) et (V), R³ et R⁴ représentent l'hydrogène ou des groupes méthyle.

6. Complexes selon une ou plusieurs des revendications 1 à 5, **caractérisés en ce que**, dans les formules générales (II), (III), (IV) et (V), R³ et R⁴ d'une part, R⁵ et R⁶ d'autre part, représentent ensemble les groupements (CH)₄, (CH₂)₄, (CH₂)₅.

7. Complexes selon une ou plusieurs des revendications 1 à 6, **caractérisés en ce que**, dans les formules générales (IV) et (V), Y représente un groupe méthylène, diméthylméthylène, diphénylméthylène ou éthylidène.

8. Complexes selon une ou plusieurs des revendications 1 à 6, **caractérisés en ce que**, dans les formules générales (IV) et (V), Y représente un groupe diméthylsilylène ou tétraméthyldisilylène.

9. Complexes selon une ou plusieurs des revendications 1 à 8, **caractérisés en ce que**, dans la formule générale (I), b est égal à 1.

10. Complexes selon une ou plusieurs des revendications 1 à 9, **caractérisés en ce que**, dans la formule générale (I), A représente un ion halogénure ou pseudo-halogénure, un ion tétraphénylborate, tétrafluoroborate, hexafluorophosphate, acétate, tétracarbonylcobaltate, hexafluoroferrate, tétrachloroferrate, tétrachloroaluminate ou tétrachloropalladate.
